(19) 

Europäisches
Patentamt
European
Patent Office
Office européen
des brevets

(11) **EP 3 639 741 A1**

(12) **EUROPEAN PATENT APPLICATION**
published in accordance with Art. 153(4) EPC

(43) Date of publication:
**22.04.2020 Bulletin 2020/17**

(21) Application number: **18817065.8**

(22) Date of filing: **15.06.2018**

(51) Int Cl.:
**A61B 5/055** (2006.01)

(86) International application number:
**PCT/JP2018/022851**

(87) International publication number:
**WO 2018/230690 (20.12.2018 Gazette 2018/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.06.2017 JP 2017118731**

(71) Applicant: **Keio University**
**Tokyo 108-8345 (JP)**

(72) Inventors:
• **HATA Junichi**
**Tokyo 160-8582 (JP)**
• **NAKASHIMA Daisuke**
**Tokyo 160-8582 (JP)**
• **NAKAMURA Masaya**
**Tokyo 160-8582 (JP)**
• **SERA Yasushi**
**Tokyo 160-8582 (JP)**

(74) Representative: **Schön, Christoph**
**Dr. Schön, Neymeyr & Partner mbB**
**Bavariaring 26**
**80336 München (DE)**

(54) **EVALUATING METHOD FOR DIFFERENTIATING BETWEEN SLOW MUSCLE AND FAST MUSCLE USING MRI**

(57) Slow muscle and fast muscle can be distinguished by using a QSI method while devising a pulse sequence system and its conditions and using mean displacement, kurtosis or probability at zero displacement as a parameter. A method for typing skeletal muscle non-invasively can be provided.

Figure 4

IMAGE EXAMPLE OF QSI TENSOR CALCULATION FOR HUMAN:
DIFFERENCE BETWEEN FAST MUSCLE
AND SLOW MUSCLE DETECTABLE

TIBIA

T2 WEIGHTED IMAGE

ANTERIOR TIBIAL MUSCLE:
FAST MUSCLE DOMINANT

SOLEUS MUSCLE:
SLOW MUSCLE DOMINANT

QSI: MAPPING IMAGE OF FA VALUE DERIVED
BY TENSOR CALCULATION OF PROBABILITY
AT ZERO DISPLACEMENT

EP 3 639 741 A1

**Description**

Technical Field

The present invention relates to a method for typing skeletal muscle by a non-invasive approach. In particular, the present invention relates to a method of evaluation for distinguishing slow muscle and fast muscle of skeletal muscles using magnetic resonance imaging (hereinafter described as MRI). The invention particularly relates to a method for analysis using q-space imaging (hereinafter described as QSI).

Background Art

Skeletal muscle fibers are generally classified into two types: slow muscle fibers and fast muscle fibers. Slow muscle is a muscle fiber which starts slowly but is highly enduring. Fast muscle is a muscle fiber capable of responding to quick movement but is less enduring. It is considered that the ratio of slow muscle to fast muscle required differs greatly depending on the type of sports. For example, the ratio of slow muscle is said to be high in marathon runners and Nordic skiers, while the ratio of fast muscle is high in powerlifting athletes and sprinters.

It is thought that the ratio between slow muscle and fast muscle depends largely on genetic factors, and which sports are suited is decided to some extent when one is born. Thus, suitable sports are determined in childhood to adolescence when people start doing sports seriously, regardless of their awareness.

Although histological examination is available for slow muscle fibers and fast muscle fibers, since biopsy is required and muscle damage is caused, usually such examination is not performed. If the mass and proportion of slow muscle and fast muscle are obtained in a non-invasive way, such scientific indexes can be a standard for determining suitable sports and selecting methods of training.

Meanwhile, activities of daily living, i.e., ADL, of elderly people are reduced due to a decrease in muscle mass. In particular, severe decrease in muscle mass called sarcopenia causes the elderly to be bedridden, and this leads to an increase in nursing care expenses, and is now a serious social problem. A muscle volume is measured at present; however, if more detailed information is available, such information helps to elucidate pathological conditions and enables appropriate preventive intervention and appropriate therapy to be provided.

Diseases which cause atrophy of the skeletal muscles are roughly classified into myogenic diseases caused by muscle itself and neurogenic diseases caused by the peripheral nerve joined to the muscle. Although differential diagnosis between the myogenic disease and the neurogenic disease has been made by biopsy, non-invasive imaging method for the skeletal muscles may be an alternative pathological examination.

MRI is a method for analysis which focuses on diffusion of water molecules to visualize highly oriented tissues. QSI is an imaging method in which data is obtained while changing b value settings in diffusion weighted imaging, hereinafter described as DWI, and the data is analyzed using Fourier transform (Non Patent Literature 1). While both DWI and QSI are a method for analysis reflecting diffusion of water molecules, analysis with DWI is based on free diffusion of water molecules and analysis with QSI is based on limited diffusion.

Water molecules do not diffuse freely in a living body, and their diffusion is restricted by restrictive structures formed of a cell membrane or collagen fiber. QSI is an approach that analyzes behavior of water molecules diffusing in a limited way, and is capable of evaluating the extent of deviation from normal distribution of water molecules. QSI can also respond to anisotropic diffusion and thus enables detailed analysis of restricted diffusion in living tissues.

QSI can measure, in micrometers, in what size of a restricted structure water molecules, which is a target of measurement, exist. As information on micro-sized structures can be obtained by QSI, this has been applied to methods for obtaining biological information in cranial nerve diseases and degenerative intervertebral discs (Non Patent Literatures 2 to 4).

Analysis of the skeletal muscles using QSI has also been reported (Non Patent Literatures 5, 6). Non Patent Literature 5 discloses analysis of the skeletal muscles of the tongue by QSI, which reveals fiber orientation. Non Patent Literature 6 discusses the possibility of QSI as to what information can be obtained and assessed by using QSI for skeletal muscle (Non Patent Literature 6). However, Non Patent Literature 5 analyzes orientation of muscle fibers, not the structure of single muscle cells. Furthermore, Non Patent Literature 6 does not describe the results of actual measurement or analysis of skeletal muscle, but only mentions the possibility of application of QSI to skeletal muscle.

Citation List

Non Patent Literature

Non Patent Literature 1: Callaghan, P. T. et al., 1991, Nature, Vol. 351, p. 467-469.

Non Patent Literature 2: Yamada, I. et al., 2015, Magn. Reson. Med., Vol. 73, p. 2262-2273.

Non Patent Literature 3: Fujiyoshi, K. et al., 2016, J. Neurosci., Vol. 36, p. 2796-2808.

Non Patent Literature 4: Nakashima, D. et al., 2017, ORS 2017 Annual Meeting, Paper No. 47.

Non Patent Literature 5: Erik, N. et al., 2015, Biophys J. 108 (11) : 2740-2749.

Non Patent Literature 6: Hata, J., 2012, INNERVISION, Vol. 27(3, p. 28-29.

Non Patent Literature 7: Stejskal, E. O. and Tanner, J. E. 1965, J. Chem. Phys. Vol. 42, p. 288-292

Non Patent Literature 8: Tanner, J. E., 1970, Vol. 52, J. Chem. Phys. p. 2523-2526.

Non Patent Literature 9: Schroder, J. M. et al., 1978, Acta Neuropathol., Vol. 43, p. 169-178.

Non Patent Literature 10: Kern, H. et al, 2014, Front. Aging Neurosci., 6:189. doi: 10.3389/fnagi.2014.00189. eCollection 2014.

Non Patent Literature 11: Kawamoto, T., 2003, Arch. Histol. Cytol., Vol. 66(2), p. 123-143.

Non Patent Literature 12: Schiaffino, S. & Reggiani, C., 2011, Phys. Rev., Vol. 91, p. 1447-1531.

## Summary of Invention

Technical Problem

An object of the present invention is to analyze fine structures of tissues in a non-invasive manner, which have conventionally been visualized by analysis such as pathological examination using a tissue. More specifically, an object of the present invention is to establish a method for evaluating the skeletal muscles non-invasively using QSI.

Since use of QSI enables analysis of information on micro-sized structures, slow muscle and fast muscle of skeletal muscles can be distinguished by a non-invasive approach. Thus, a method for judging suitable sports and a method for evaluating training methods in sports medicine can be provided. Furthermore, a method for assessing not only properties of the skeletal muscles of human but also, for example, quality of the leg of racehorses, can be provided.

Moreover, this non-invasive method using MRI is not a burden on a patient even when the same patient undergoes examination repeatedly. Thus, since follow-up examination becomes available, not only diseases such as sarcopenia and skeletal muscle diseases can be distinguished, but also an objective standard can be given in, for example, assessing therapeutic methods.

Solution to Problem

The present invention relates to a method for examining skeletal muscle using MRI, and a program.

(1) A method for examining skeletal muscle using MRI, comprising measuring a diameter of a muscle fiber using Q-space imaging (QSI).

(2) The method according to (1), wherein a cell diameter of the muscle fiber reflects a difference between slow muscle and fast muscle and a slow muscle fiber and a fast muscle fiber are distinguished.

(3) The method according to (1) or (2), wherein an imaging method of MRI is a diffusion weighted stimulated echo (DW STE) method.

(4) The method according to (3), wherein imaging in the DW STE method is performed while setting a TE (echo time) short and extending a diffusion time.

(5) The method according to (3) or (4), comprising:

collecting data while extending the diffusion time in the DW STE method in a multi-step manner up to a diffusion time longer than that (200 ms) sufficient for a structure of skeletal muscle to be defined (about 1000 ms) ;

visualizing a structure for exchanging water molecules of a cell membrane, which is specific to a fast muscle fiber and formed of aquaporin (AQP) 4 protein; and

visualizing a slow muscle fiber and a fast muscle fiber by the visualization of a structure for exchanging water molecules.

(6) The method according to any one of (1) to (5), wherein mean displacement, kurtosis or probability at zero displacement is used as a parameter in the QSI.

(7) The method according to any one of (1) to (6), wherein in the QSI, tensor calculation is performed while incorporating an idea of vector, thereby obtaining a $\lambda 1$ value (axial direction (AD)), a $\lambda 2$ value, a $\lambda 3$ value (radial direction (RD)), a fractional anisotropy value (FA) and a mean values (MD) of a QSI parameter: mean displacement, kurtosis or probability at zero displacement to make determination.

(8) A method for examination, wherein the method according to any one of (1) to (7) is used for determining a suitable sport, assessing sarcopenia, or assessing quality of the leg of a racehorse.

(9) A method for examination using MRI, comprising: using QSI; and performing tensor calculation while incorporating an idea of vector of a QSI parameter, wherein the method is used for analyzing a biological structure.

(10) A program executed by a computer, comprising: a step of extracting a spectrum based on a b-value, axial information and a diffusion time using MRI equipment based on data in each voxel of a target obtained by a DW-STE method and performing an operation using at least one member of a $\lambda 1$ value (axial direction (AD)), a $\lambda 2$ value, a $\lambda 3$ value (radial direction (RD)), a fractional anisotropy value (FA) and a mean value (MD) of mean displacement, kurtosis or probability at zero displacement in a QSI analysis; and

a step of quantitatively describing a condition of a target based on a result of the operation.

(11) The program according to (10), wherein the step of describing the result of the operation as an image is executed by a computer.

(12) The program according to (10) or (11), wherein the target is skeletal muscle.

The skeletal muscle can be assessed while distinguishing slow muscle and fast muscle by a non-invasive method, i.e., MRI examination. Since use of QSI enables analysis of information on micro-sized structures to assess skeletal muscle, more information becomes available than by conventional methods even by a non-invasive examination. Information comparable to, or more than, that obtained by biopsy can be obtained in the field such as sports medicine and skeletal muscle diseases, where biopsy has not been done due to a heavy burden on patients.

Brief Description of Drawings

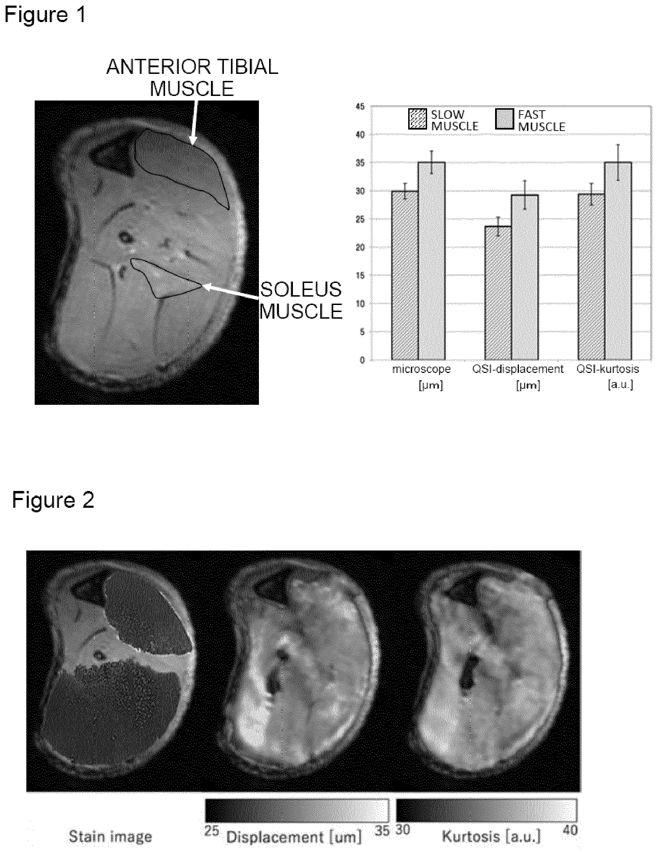

[Figure 1] Figure 1 shows a view comparing histological observation and QSI parameters in animal experiment.

[Figure 2] Figure 2 shows a view comparing histological observation and QSI parameters superposed on images in animal experiment.

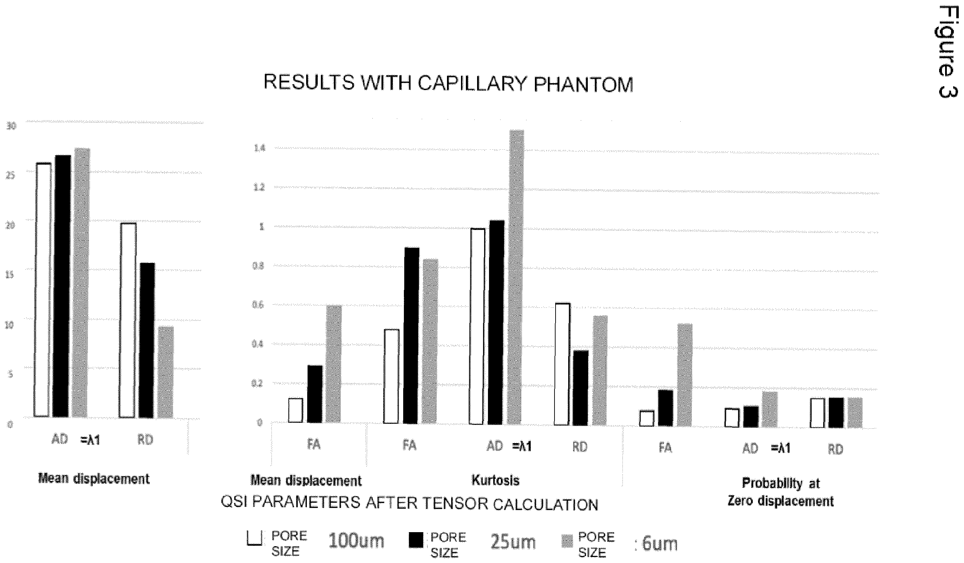

[Figure 3] Figure 3 shows a view comparing values of QSI parameters after tensor calculation using three types of capillary phantoms with a known pore size (microchannel plates: pore size 100 $\mu$m, 25 $\mu$m, 6 $\mu$m). At AD (= $\lambda 1$), for AD, RD, and FA of mean displacement, AD of kurtosis, and FA of probability at zero displacement, the difference of the three is clear.

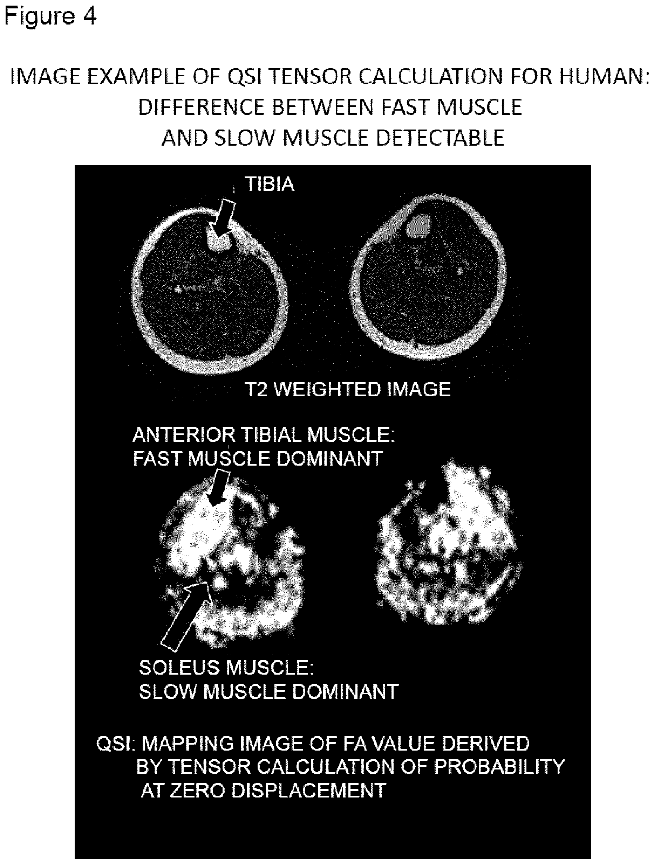

[Figure 4] Figure 4 shows an image example of QSI tensor calculation for a human. In the T2 weighted image, the difference in the dark area is not clear and no difference is observed in muscular portions. By contrast, when QSI is used, the difference between the anterior tibial muscle: fast muscle dominant and the soleus muscle: slow muscle dominant can be clearly distinguished.

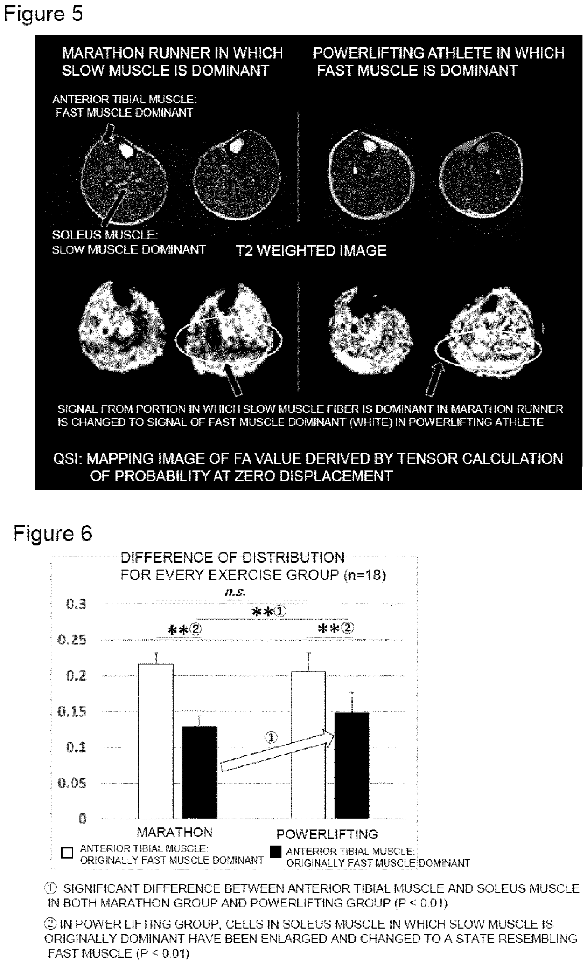

[Figure 5] Figure 5 shows an image example of QSI tensor calculation for a human. The difference between a marathon runner with a high ratio of slow muscle and a powerlifting athlete with a high ratio of fast muscle is compared. In the powerlifting athlete, the signal value from the portion of slow muscle fiber in the soleus muscle in the marathon runner has been changed to a signal value similar to that from fast muscle fiber in the anterior tibial muscle. This shows that slow muscle has been converted into fast muscle in the powerlifting athlete.

[Figure 6] Figure 6 shows a bar graph showing the difference of the respective muscles of Figure 5. The figure shows that there is a significant difference (p < 0.01) between the portion in which fast muscle fibers are dominant (anterior tibial muscle) and the portion in which slow muscle fibers are dominant (soleus muscle) in both the marathon runner and the powerlifting athlete. Furthermore this shows that, in the powerlifting athlete, the portion in which slow muscle fiber is dominant is significantly converted into fast muscle (p < 0.01).

Description of Embodiments

As described above, methods for analyzing biological information by QSI have been reported in the assessment of nerve tract of the cranial nerve system, the analysis of demyelination or tissue types of malignant tumors, and the assessment of degenerative intervertebral discs (Non Patent Literatures 2-4). However, distinction and detection of slow muscle and fast muscle of the skeletal muscle was impossible even by collecting signals and analyzing parameters in the same manner as in the region of the cranial nerve system or intervertebral discs.

Then, to apply QSI to skeletal muscle, the present inventors have conducted intensive studies on a pulse sequence system and its conditions. As a result, it has been found that a pulse sequence system and its conditions different from those for the region of the central nerve system or intervertebral discs need to be used in order to distinguish and assess slow muscle and fast muscle of skeletal muscles.

For diffusion measurement, mainly two approaches of: Diffusion Weighted Spin Echo (DW SE method) (Non Patent Literature 7), which is one of the Pulsed Gradient Spin Echo methods (PGSE methods), and Diffusion Weighted Stimulated Echo (DW STE method) (Non Patent Literature 8) have been used.

Since the signal intensity in the DW SE method is twice that in the DW STE method, usually the DW SE method is used. Thus, the DW SE method is used in almost all systems. Since the skeletal muscles have very small NMR relaxation characteristics (T2-value) of 30 ms, attenuation of signals is significant in the DW SE imaging method, and thus good diffusion measurement is difficult. Meanwhile, in the DW STE imaging method, the signal intensity in measurement is higher when NMR relaxation characteristics of the target tissue are T1 >> T2, and thus the DW STE imaging method is a more advantageous pulse sequence system for skeletal muscle than a DW SE pulse sequence system.

Skeletal muscle cells have a cell diameter of about 30 - 50 $\mu$m, which is much larger than the cell diameter of cells in the region of the central nerve, which is about a few micrometers (Non Patent Literatures 9, 10). Thus, transfer of water molecules in skeletal muscle cells caused by diffusion is very different from that in nerve cells.

A mean-square displacement, MSD, r by diffusion based on the Brownian motion is represented by the following equation 1.

[Equation 1]

$$<r>^2 = 6 \; Dt \; (t = \text{time of transfer of particle}, \; D = \text{diffusion coefficient})$$

When the diffusion time for accurately assessing skeletal muscle cells using QSI is theoretically calculated by this equation, a diffusion time of about 100 to 200 ms is determined to be necessary. In the DW STE method, a very long diffusion time can be established by setting a TE (echo time) short and extending the diffusion time (also referred to as mixing time or interval time). In other words, in the DW STE method, a very long diffusion time can be established while maintaining the intensity of signals obtained.

To obtain information on the difference in the motor function of the skeletal muscle by MRI, we have selected a DW STE method, a QSI pulse sequence system which was conventionally unavailable; and for its conditions, we have devised to shorten TE and extend the diffusion time to obtain signals, and have obtained signals in those conditions.

A computer program executes all steps of analysis according to a QSI method based on the data obtained by MRI. More specifically, a computer executes operation of extracting b-value data, axial information and diffusion times and calculating $\lambda 1$ values (axial direction (AD)), $\lambda 2$ values, $\lambda 3$ values (radial direction (RD)), fractional anisotropy values (FA) and mean values (MD) of mean displacement, kurtosis or probability at zero displacement, based on the data in each voxel of the target skeletal muscle obtained by MRI equipment. Furthermore, conditions of skeletal muscle can be quantitatively shown in numerical values, or can be visualized at its location by a method such as color mapping, based on the results of operation. The data can be displayed in not only two-dimensional images but also three-dimensional images, which are easier to understand.

A method usually employed in this field is used as the method of calculation of the respective values executed by a computer (e.g., Non Patent Literatures 1, 2). In a QSI method, a signal attenuation curve is derived from signal values of a few steps in the respective vector directions, and inverse Fourier transform is performed to create probability mutation probability distribution. Then, mean displacement, kurtosis and probability at zero displacement are calculated, and tensor calculation is performed to calculate $\lambda 1$ values (axial direction (AD)), $\lambda 2$ values, $\lambda 3$ values (radial direction (RD)), fractional anisotropy values (FA) and mean values (MD) of them.

Tensor calculation of QSI parameters, which was not reported before, has been first discovered by the present inventors to be applicable to the differentiation of the skeletal muscle. This can be applied to not only the skeletal muscle but also biological structures with a restrictive structure similar to that of the skeletal muscle, such as nerve cells, epithelium cells such as intestinal tract cells, liver cells, intervertebral discs and extracellular matrix such as cartilage. Furthermore, as described in the following measurement examples, pore sizes of small pores can also be measured in addition to measurement of living bodies, and thus the method is industrially applicable.

The present invention will be described in detail with reference to data.

It has been known from analysis of stained tissue images that mouse anterior tibial muscle is the skeletal muscle rich in fast muscle and mouse soleus muscle is rich in slow muscle. The image on the left in Figure 1 shows an MRI T1 weighted image showing a horizontal section of the calf of the hind leg of a mouse. As shown in the left of Figure 1, the conventional MRI T1 weighted image was incapable of showing a difference between slow muscle and fast muscle. Then, to be able to distinguish slow muscle and fast muscle in this region, the muscle was analyzed while devising settings of MRI as described above.

Using 7 tesla MRI (Biospec 70/16 MRI; Bruker BioSpin Ettlingen) as MRI equipment, a mouse (C57BL/6J, male, 12 weeks old) anesthetized with a mixture of 2.0% isoflurane (Abbot Laboratories), oxygen and air, was imaged in a prone position in the following imaging conditions.

Method and conditions of imaging: DW STE method, repetition time (TR)/ echo time (TE), 4000 / 12.6 ms; Δ/δ, 101.2 / 3.6 ms; input max b-value, 0-4000 s/ mm$^2$; field of view (FOV), 100 × 100 μm$^2$; matrix size, 160 × 160; slice thickness, 1 mm; motion probing gradient (MPG) moment, two axes (x and y).

As the cell diameter of fast muscle is larger than that of slow muscle, settings were devised as described above so that the difference can be detected. In other words, the differentiation between fast muscle and slow muscle described below corresponds to an assessment method of the cell diameter of muscle cells.

The graph on the right of Figure 1 shows a comparison between a histological observation and results obtained by QSI for mouse anterior tibial muscle and soleus muscle. For the histological observation, the same mouse used for imaging by MRI was perfusion fixed, and a frozen section was prepared by the Kawamoto method (Non Patent Literature 11).

The frozen section was fixed with 4% paraformaldehyde at room temperature for 15 minutes and post-fixation with methanol at -20°C for 10 minutes was performed, and immunostained by a routine method. Monoclonal antibody BA-D5 (antibody specific to myosin heavy-chain type I, DSHB Concentrate), SC-71 (antibody specific to myosin heavy-chain type IIA, DSHB Concentrate) and BF-F3 (antibody specific to myosin heavy-chain type IIB, DSHB Concentrate) were used as primary antibodies, and utilizing different isotypes of the respective monoclonal antibodies BA-D5, SC-71, BF-F3, the same section was triple-stained to distinguish slow muscle fiber (type I fiber) and fast muscle fiber (type IIa, b fiber), and the muscle was observed with a microscope to measure the cell diameters of slow muscle fiber and fast muscle fiber.

The results of investigation of various parameters of QSI method show that the difference between slow muscle and fast muscle can be detected when mean displacement, kurtosis and probability at zero displacement were used as parameters (right in Figure 1). The difference in cell diameter of slow muscle and fast muscle can be distinguished to the same extent as in tissue staining, even by using either parameter.

Furthermore, when the mean displacement is used as a parameter, the cell diameter of muscle fiber can be determined. When a different equipment is used, values obtained by analysis cannot be compared or examined depending on the parameter. However, the mean displacement can be calculated as a universal parameter independent of equipment, values obtained in different facilities can be mutually compared and analyzed.

Figure 2 shows T1 weighted images and values obtained by analysis superposed thereon. It is clear that there is a good agreement between the image obtained after staining (left in Figure 2) and the results obtained with mean displacement (center in Figure 2) and kurtosis (right in Figure 2) in QSI. Conventionally, it was impossible to distinguish slow muscle and fast muscle, which have a different motor function, by analysis with MRI. According to the method described in the present Example, the difference between slow muscle and fast muscle is regarded as a difference in the cell diameter of muscle fiber, enabling imaging while distinguishing the two.

When the results with animals described above are applied to humans, a method with higher sensitivity is preferred. The following method for visualizing a functional difference of aquaporin 4 (AQP4) and tensor calculation enable more detailed analysis using QSI.

AQP4, a cell membrane protein, is strongly expressed in the fibrous membrane of glycolytic fast muscle fibers and oxidative-glycolytic fast muscle fibers (fast muscle fibers) among the muscle fibers, and facilitates flow of water molecules into cells (Non Patent Literature 12). AQP4 protein enables quick exchange of water molecules between the inside and the outside of cells. When this functional difference is reflected into a contrast, the difference of the function of AQP4, i.e., the difference between fast muscle fibers and slow muscle fibers can be distinguished. To reflect this difference into a contrast, data must be collected while extending the diffusion time in the DW STE method described above in a multi-step manner up to a diffusion time longer than that (200 ms) sufficient for the structure of skeletal muscle to be defined (about 1000 ms).

Furthermore, tensor calculation while incorporating an idea of vector into the above QSI, which was not existing before, can provide values at sensitivity higher than that with usual QSI parameters (mean displacement, kurtosis, probability at zero displacement). More specifically, $\lambda 1$ values (axial direction (AD)), $\lambda 2$ values, $\lambda 3$ values (radial direction (RD)), fractional anisotropy values (FA) and mean values (MD) of usual QSI parameters (mean displacement, kurtosis, probability at zero displacement) can be obtained. The difference between slow muscle and fast muscle can be determined as the difference in cell diameter. It has been shown that analysis using the above values can distinguish the difference in cell diameter, i.e., properties of muscle, in detail.

Furthermore, tensor calculation while incorporating an idea of vector into the above QSI, which was not existing before, can provide values at sensitivity higher than that with usual QSI parameters (mean displacement, kurtosis, probability at zero displacement). More specifically, $\lambda 1$ values (the same as axial diffusivity (AD)), $\lambda 2$ values, $\lambda 3$ values, fractional anisotropy (FA) values, mean diffusivity (MD) values and radial diffusivity (RD) values of usual QSI parameters (mean displacement, kurtosis, probability at zero displacement) can be obtained. The difference between slow muscle and fast muscle can be determined as the difference in cell diameter. It has been shown that analysis using the above values can distinguish the difference in cell diameter, i.e., properties of muscle, in detail.

First, the results of analysis using porous phantoms with a known restrictive structure will be shown. The results of a comparison of QSI parameter values after tensor calculation using three types of capillary phantoms (microchannel plates: pore size 100 $\mu$m, 25 $\mu$m, 6 $\mu$m) are shown in Table 1 and Figure 3. If QSI parameters increase or decrease depending on the cell diameter, it is highly likely that the method of analysis reflects the size of the restrictive structure. As shown in Figure 3, a correlation is found between the pore size and QSI parameters for AD, RD, FA of mean displacement, AD of kurtosis, and FA, AD (= $\lambda$1) of probability at zero displacement. The difference in the size of three pores is clear, suggesting the possibility that these parameters are useful for analysis of humans.

[Table 1]
Results with capillary phantom

| | | | Pore size | | |
| --- | --- | --- | --- | --- | --- |
| | | | 100um | 25um | 6um |
| QSI parameters after tensor calculation | **Mean displacement** | FA | 0.123724722 | 0.28984337 | 0.594497093 |
| | | AD =$\lambda$1 | 25.87332398 | 26.62446603 | 27.35251131 |
| | | RD | 19.88563845 | 15.70761713 | 9.355988883 |
| | **Kurtosis** | FA | 0.475572948 | 0.898114836 | 0.837748603 |
| | | AD =$\lambda$1 | 0.996474831 | 1.040182273 | 2.048867063 |
| | | RD | 0.615750083 | 0.379696737 | 0.561332528 |
| | **Probability at zero displacement** | FA | 0.083142914 | 0.186816174 | 0.525306688 |
| | | AD =$\lambda$1 | 0.090688948 | 0.107835315 | 0.182074347 |
| | | RD | 0.150133849 | 0.152625458 | 0.154649493 |

All in arbitrary units [a.u.]

Next, the results of analysis of human subjects will be described (Figure 4). The human lower leg was analyzed according to a usually used T2 weighted image and QSI. Figure 4 shows a vertical section of the lower leg of a human. In the T2 weighted image on the top, the difference in the dark area is not clear and no difference is observed in muscular portions. By contrast, in the analysis using QSI on the bottom, the difference between the anterior tibial muscle in which fast muscle is dominant and the soleus muscle in which slow muscle is dominant could be clearly distinguished.

The same analysis was performed for a marathon runner with a high ratio of slow muscle and a powerlifting athlete with a high ratio of fast muscle (Figure 5). A T2 weighted image is shown on the top and an FA mapping image according to QSI is shown on the bottom. According to the analysis using QSI, in the powerlifting athlete, the signal value from the portion of slow muscle fiber in the soleus muscle in the marathon runner has been changed to a signal value similar to that from fast muscle fiber in the anterior tibial muscle. In other words, in the case of the powerlifting athlete, the diameter of muscle fiber has been increased even in the region of the soleus muscle, in which slow muscle is dominant, and thus slow muscle has been converted into fast muscle.

Figure 6 shows quantification of the results of the images shown in Figure 5. The figure shows that there is a significant difference (p < 0.01) between the portion in which fast muscle fibers are dominant (anterior tibial muscle) and the portion in which slow muscle fibers are dominant (soleus muscle) in both the marathon runner and the powerlifting athlete. Furthermore, in the powerlifting athlete, the portion in which slow muscle fibers are dominant has been significantly enlarged and converted into fast muscle (p < 0.01) compared with the marathon runner.

Unlike histological examination in which only a portion is examined, MRI examination is capable of displaying tomographic images of any angle, and thus the target can be assessed three-dimensionally. Therefore, the amount of information to be obtained is significantly increased compared with conventional methods of tissue staining.

Conventionally, only invasive pathological examination was available for examination of the skeletal muscles, but the present method allows examination by non-invasive MRI, and thus even if examination is repeated over time, it is not burden on a patient. Accordingly, an increase in muscle mass caused by rehabilitation and an increase in muscle mass caused by sports can be observed with time. Furthermore, more detailed information, for example, which of slow muscle and fast muscle has been increased, can be obtained.

Moreover, this method of analysis detects the thickness of a muscle fiber, and thus conversion of, for example, muscle power into a value is available for athletes. Thus, results of training can be defined as an objective value.

Industrial Applicability

Slow muscle and fast muscle of the skeletal muscle can be distinguished and change in muscle mass can be analyzed by a non-invasive examination. Thus, the skeletal muscle can be objectively assessed in sports medicine, distinction of skeletal muscle disease, and increase and decrease of muscle mass in sarcopenia, rehabilitation and the like.

Furthermore, various troubles of muscle such as sore muscle, which are caused by training, may also be assessed as an objective value. The time of resuming training after troubles, for example, has been determined from symptoms or based on experience. However, since conditions of skeletal muscle will be able to be judged as an objective value, phenomena which have conventionally been assumed from symptoms can be judged by objective values and images.

Change in muscle fibers caused by microneuropathy will be able to be grasped, which was unable to be detected before, such as change in paraspinal muscle and psoas muscle fibers associated with neuropathy in intervertebral discs or around the joint involving lower back pain or joint pain. The above can also be applied to assessment of animals with varying qualities of the leg, such as racehorses as well as humans.

**Claims**

1. A method for examining skeletal muscle using MRI, comprising measuring a diameter of a muscle fiber using Q-space imaging (QSI).

2. The method according to claim 1, wherein a cell diameter of the muscle fiber reflects a difference between slow muscle and fast muscle and a slow muscle fiber and a fast muscle fiber are distinguished.

3. The method according to claim 1 or 2, wherein an imaging method of MRI is a diffusion weighted stimulated echo (DW STE) method.

4. The method according to claim 3, wherein imaging in the DW STE method is performed while setting a TE (echo time) short and extending a diffusion time.

5. The method according to claim 3 or 4, comprising:

   collecting data while extending the diffusion time in the DW STE method in a multi-step manner up to a diffusion time longer than that (200 ms) sufficient for a structure of skeletal muscle to be defined (about 1000 ms) ;
   visualizing a structure for exchanging water molecules of a cell membrane, which is specific to a fast muscle fiber and formed of aquaporin (AQP) 4 protein; and
   visualizing a slow muscle fiber and a fast muscle fiber by the visualization of a structure for exchanging water molecules.

6. The method according to any one of claims 1 to 5, wherein mean displacement, kurtosis or probability at zero displacement is used as a parameter in the QSI.

7. The method according to any one of claims 1 to 6, wherein in the QSI, tensor calculation is performed while incorporating an idea of vector, thereby obtaining a $\lambda 1$ value (axial direction (AD)), a $\lambda 2$ value, a $\lambda 3$ value (radial direction (RD)), a fractional anisotropy value (FA) and a mean values (MD) of a QSI parameter: mean displacement, kurtosis or probability at zero displacement to make determination.

8. A method for examination, wherein the method according to any one of claims 1 to 7 is used for determining a suitable sport, assessing sarcopenia, or assessing quality of the leg of a racehorse.

9. A method for examination using MRI, comprising:

   using QSI; and
   performing tensor calculation while incorporating an idea of vector of a QSI parameter,
   wherein the method is used for analyzing a biological structure.

10. A program executed by a computer, comprising:

a step of extracting a spectrum based on a b-value, axial information and a diffusion time using MRI equipment based on data in each voxel of a target obtained by a DW-STE method and performing an operation using at least one member of a $\lambda 1$ value (axial direction (AD)), a $\lambda 2$ value, a $\lambda 3$ value (radial direction (RD)), a fractional anisotropy value (FA) and a mean value (MD) of mean displacement, kurtosis or probability at zero displacement in a QSI analysis; and

a step of quantitatively describing a condition of a target based on a result of the operation.

11. The program according to claim 10, wherein the step of describing the result of the operation as an image is executed by a computer.

12. The program according to claim 10 or 11, wherein the target is skeletal muscle.

Figure 1

Figure 2

Stain image  25 Displacement [um] 35 30  Kurtosis [a.u.]  40

RESULTS WITH CAPILLARY PHANTOM

QSI PARAMETERS AFTER TENSOR CALCULATION

Figure 3

EP 3 639 741 A1

# Figure 4

IMAGE EXAMPLE OF QSI TENSOR CALCULATION FOR HUMAN:
DIFFERENCE BETWEEN FAST MUSCLE
AND SLOW MUSCLE DETECTABLE

## Figure 5

MARATHON RUNNER IN WHICH SLOW MUSCLE IS DOMINANT

POWERLIFTING ATHLETE IN WHICH FAST MUSCLE IS DOMINANT

ANTERIOR TIBIAL MUSCLE: FAST MUSCLE DOMINANT

SOLEUS MUSCLE: SLOW MUSCLE DOMINANT

T2 WEIGHTED IMAGE

SIGNAL FROM PORTION IN WHICH SLOW MUSCLE FIBER IS DOMINANT IN MARATHON RUNNER IS CHANGED TO SIGNAL OF FAST MUSCLE DOMINANT (WHITE) IN POWERLIFTING ATHLETE

QSI: MAPPING IMAGE OF FA VALUE DERIVED BY TENSOR CALCULATION OF PROBABILITY AT ZERO DISPLACEMENT

## Figure 6

DIFFERENCE OF DISTRIBUTION FOR EVERY EXERCISE GROUP (n=18)

MARATHON POWERLIFTING

☐ ANTERIOR TIBIAL MUSCLE: ORIGINALLY FAST MUSCLE DOMINANT

■ ANTERIOR TIBIAL MUSCLE: ORIGINALLY FAST MUSCLE DOMINANT

① SIGNIFICANT DIFFERENCE BETWEEN ANTERIOR TIBIAL MUSCLE AND SOLEUS MUSCLE IN BOTH MARATHON GROUP AND POWERLIFTING GROUP (P < 0.01)

② IN POWER LIFTING GROUP, CELLS IN SOLEUS MUSCLE IN WHICH SLOW MUSCLE IS ORIGINALLY DOMINANT HAVE BEEN ENLARGED AND CHANGED TO A STATE RESEMBLING FAST MUSCLE (P < 0.01)

## INTERNATIONAL SEARCH REPORT

| International application No. |
|---|
| PCT/JP2018/022851 |

A. CLASSIFICATION OF SUBJECT MATTER
Int.Cl. A61B5/055 (2006.01)i
According to International Patent Classification (IPC) or to both national classification and IPC

B. FIELDS SEARCHED

Minimum documentation searched (classification system followed by classification symbols)
Int.Cl. A61B5/055, G01R33/20-33/64

Documentation searched other than minimum documentation to the extent that such documents are included in the fields searched
| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
MEDLINE (JDreamIII), 医中誌 WEB (Ichushi WEB)

C. DOCUMENTS CONSIDERED TO BE RELEVANT

| Category* | Citation of document, with indication, where appropriate, of the relevant passages | Relevant to claim No. |
|---|---|---|
| X<br>A | 畑純一, q-space imaging による評価の可能性, INNERVISION, 2012, vol. 27, no. 3, pp. 28-29, non-official translation (HATA, Junichi, "Feasibility of evaluation by q-space imaging") | 1-3, 6, 8<br>4-5, 7, 9-12 |
| A | BASSER, Peter J., "Inferring Microstructural Features and the Physiological State of Tissues from Diffusion-Weighted Images", NMR IN BIOMEDICINE, 1995, vol. 8, pp. 333-344 | 1-12 |
| A | HATA, Junichi et al., "Diffusion Fractional Anisotropy-based Transformation in Skeletal MuscleCaused by Pressure", Magn Reson Med Sci, 2012, vol. 11, no. 3, pp. 179-184 | 1-12 |
| A | US 2014/0357979 A1 (BASSER, Peter J.) 04 December 2014, entire text, all drawings & WO 2013/062684 A1 | 1-12 |

☐ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26 July 2018 (26.07.2018) | 14 August 2018 (14.08.2018) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
|---|---|

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CALLAGHAN, P. T. et al.** *Nature,* 1991, vol. 351, 467-469
- **YAMADA, I. et al.** *Magn. Reson. Med.,* 2015, vol. 73, 2262-2273
- **FUJIYOSHI, K. et al.** *J. Neurosci.,* 2016, vol. 36, 2796-2808
- **NAKASHIMA, D. et al.** *ORS 2017 Annual Meeting,* 2017, (47
- **ERIK, N. et al.** *Biophys J.,* 2015, vol. 108 (11), 2740-2749
- **HATA, J.** *INNERVISION,* 2012, vol. 27 (3), 28-29
- **STEJSKAL, E. O. ; TANNER, J. E.** *J. Chem. Phys.,* 1965, vol. 42, 288-292
- **TANNER, J. E.** *J. Chem. Phys.,* 1970, vol. 52, 2523-2526
- **SCHRODER, J. M. et al.** *Acta Neuropathol.,* 1978, vol. 43, 169-178
- **KERN, H. et al.** *Front. Aging Neurosci.,* 2014, vol. 6, 189
- **KAWAMOTO, T.** *Arch. Histol. Cytol.,* 2003, vol. 66 (2), 123-143
- **SCHIAFFINO, S. ; REGGIANI, C.** *Phys. Rev.,* 2011, vol. 91, 1447-1531